# EUROPÄISCHE PATENTSCHRIFT

(11) **EP 1 513 892 B1**
(45) Veröffentlichungstag und Bekanntmachung des Hinweises auf die Patenterteilung: **29.10.2008**
(21) Anmeldenummer: 03732501.6
(22) Anmeldetag: 30.05.2003
(51) Int. Cl.: C08K 5/20, C07C 231/02

(54) **UMSETZUNGSPRODUKTE AUS MISCHUNGEN LANGKETTIGER FETTSÄUREN UND ALIPHATISCHEN DIAMINEN UND DEREN VERWENDUNG**
CONVERSION PRODUCTS OF MIXTURES OF LONG-CHAINED FATTY ACIDS AND ALIPHATIC DIAMINES, AND THE USE THEREOF
PRODUITS DE TRANSFORMATION OBTENUS A PARTIR DE MELANGES D'ACIDES GRAS A CHAINE LONGUE ET DE DIAMINES ALIPHATIQUES, ET LEUR UTILISATION

(30) Priorität: 05.06.2002 DE 10224847
(43) Veröffentlichungstag der Anmeldung: 16.03.2005
(73) Patentinhaber: Clariant Produkte (Deutschland) GmbH, 65929 Frankfurt am Main (DE)
(72) Erfinder: HEINRICHS, Franz-Leo, 86456 Gablingen (DE); STALMANN, Ernst, Rudolf, 86153 Augsburg (DE); PECHLER, Norbert, 86368 Gersthofen (DE)
(86) Internationale Anmeldenummer: PCT/EP2003/005670
(87) Internationale Veröffentlichungsnummer: WO 2003/104318

(56) Entgegenhaltungen:
- WO-A-00/68329
- DE-A- 2 730 175
- DE-A- 19 929 962
- DE-B- 1 096 603
- DE-C- 932 965
- DE-C- 934 767
- GB-A- 677 935
- US-A- 2 901 370

## Beschreibung

Die Erfindung betrifft Umsetzungsprodukte aus Mischungen langkettiger Fettsäuren und aliphatischen Diaminen und deren Verwendung

Durch die erhebliche Zunahme des Straßenverkehrs, insbesondere des Schwerlastverkehrs, ist die Modifizierung von Bitumen (Asphalt) zur Verminderung von Straßenschäden heutzutage eine Notwendigkeit.

Zur Modifizierung von Bitumen werden hochmolekulare Verbindungen eingesetzt wie Styrol-Butadien-Styrol (SBS), amorphes Polyalphaolefin (APAO), Polyethylen (PE) oder andere Polymere oder niedermolekulare Verbindungen wie Montanwachs, Fischer-Tropsch Wachs, Amidwachse oder anorganische Modifizierungsmittel wie hydratisiertes Carbonatgestein.

Polymermodifiziertes Bitumen zeichnet sich durch eine verbesserte Kälteflexibilität, etwas erhöhtem Erweichungspunkt und gering höherer Härte gegenüber reinem Bitumen aus. Die Viskosität von polymermodifiziertem Bitumen bei Misch-, Verlege- und Verdichtungstemperatur ist aber wesentlich höher als bei nichtmodifiziertem Bitumen. Dadurch ist die Verdichtungswilligkeit bzw. das Verdichtungsvermögen des von polymermodifizierten Bitumens vermindert und der Hohlraumgehalt des Bitumens wird erhöht, was zur Reduzierung der Stabilität in der Asphaltschicht führt.

Niedermolekulare Zusätze wie Montanwachs, Fischer-Tropsch-Paraffine und andere reduzieren die Viskosität und verbessern die Verdichtungswilligkeit des Bitumens. Die Erweichungstemperatur des Bitumens wird in Abhängigkeit vom Schmelzpunkt des Modifizierungsmittels leicht erhöht, die Kälteflexibilität wird aber erheblich reduziert, mit dem Nachteil von erhöhten Kältebruchtemperaturen. Dies ist besonders bei Minus-Temperaturen, die über einen längeren Zeitpunkt auftreten, von Bedeutung.

Die Gebrauchs- und Verarbeitungseigenschaften von Bitumen für Straßenbauasphalte sind weitgehend von der Härte, dem Erweichungspunkt, der Viskosität und dem Kältebruch des jeweiligen Bitumens abhängig.

Für gute Gebrauchs- und Verarbeitungseigenschaften ist eine möglichst breite Plastizitätsspanne des Bitumens notwendig. Als Plastizitätsspanne wird die Differenz zwischen dem Erweichungspunkt Ring/Kugel nach DIN 52011/EN 1427 und dem Kältebruch nach Fraaß (DIN 52012/EN 12593) bezeichnet.

Die nachfolgende Tabelle 1 gibt einen Überblick über die Plastizitätsspanne von Bitumen B80 mit verschiedenen Zusätzen.

**Tabelle 1: Plastizitätsspanne**

| Bitumen Typ | Zusätze | Menge (Gew.-%) | Plastizitätsspanne |
|---|---|---|---|
| Bitumen B80 | kein Zusatz | 0 | -15 ...+50 °C |
| Bitumen B80 | SBS | 4 % | -20 ...+65°C |
| Bitumen B80 | Montanwachs | 3% | 0 ...+55°C |
| Bitumen B80 | Fischer Tropsch | 3% | -6 ...+75°C |
| Bitumen B80 | Amidwachs | 3 % | -11 ...+95°C |

Die Qualität des Asphalts wird immer vom schwächsten Eigenschaftsbild geprägt.

Ein Fortschritt gegenüber dem Einsatz von Polymeren oder Fischer-Tropsch-Paraffinen konnte durch die Bitumenmodifizierung mit Amidwachsen erreicht werden. Amidwachse sind Umsetzungsprodukte aus Ethylendiamin und gehärteter Talgfettsäure (siehe hierzu DE-A-2 730 175, DE-A-19 929 962 und WO-A-00/68329).

Kommerziell verfügbare Amidwachs für den Straßenbau ist ein ebenfalls ein Umsetzungsprodukt aus Ethylendiamin mit gehärteter Talgfettsäure. Talgfettsäure wird aus Talg gewonnen. Es ist eine Mischung von Fettsäuren in der Zusammensetzung

**Tabelle 2: Zusammensetzung von Talgfettsäuren (Angaben in Gew.-%)**

| Fettsäure | ungehärtet | gehärtet |
|---|---|---|
| Myristinsäure | 1-7 | 1-7 |
| Palmitinsäure | 20-35 | 20-35 |
| Stearinsäure | 15-30 | 65-80 |
| Ölsäure | 20-50 | < 2 |

Molekulare Wechselwirkungen zwischen dem Bitumen und dem Amidwachs bei höheren Temperaturen ( > 100°C) erniedrigen die Viskosität des Bitumens im Asphalt. Dadurch wird die Verarbeitbarkeit gegenüber nichtmodifiziertem Bitumen verbessert. Sinkt die Temperatur im verarbeiteten Asphalt unter 100°C, so steigt die Viskosität an und die Asphaltschicht ist schon bei höheren Temperaturen belastbar. Durch diesen Effekt kann die Bildung von Spurrinnen bei erhöhter Temperatur stark zurückgedrängt werden und die Lebensdauer der Asphaltschicht wird erhöht. Gleichzeitig kann auch weicheres Bitumen eingesetzt werden, da durch den Zusatz von Amidwachs die Härte des Bitumens zunimmt.

Nachteil dieser Modifizierung ist, dass die Kälteflexibilität des modifizierten Bitumens gegenüber nichtmodifiziertem oder polymermodifiziertem Bitumin abnimmt. So liegen die Kältebruchwerte nach Fraaß für verschiedene im Markt angebotene Produkte bei -10 bis -13°C bzw. -10 bis -11°C oder sogar bei nur -6 bis -8°C. Solche Bitumen sind für den Dauereinsatz bei tieferen Temperaturen nicht geeignet.

Es war daher die Aufgabe der vorliegenden Erfindung, ein Modifizierungsmittel für Bitumen zu finden, das die positiven Eigenschaften des kommerziellen Amidwachses zeigt ohne dabei gleichzeitig die Kälteeigenschaften des Bitumens zu verschlechtern. Gelöst wird diese Aufgabe gemäß Anspruch 1 durch Umsetzungsprodukte aus Mischungen langkettiger Fettsäuren und aliphatischen Diaminen mit einer Alkalizahl < 10 und einer Säurezahl < 15.

Das Verhältnis von Mischungen der langkettigen Fettsäuren zu aliphatischen Diaminen beträgt 2 zu 1.

Bevorzugt enthält die Mischung langkettiger Fettsäuren

| | |
|---|---|
| 0 - 7 Gew.-% | Myristinsäure |
| 0 - 85 Gew.-% | Palmitinsäure |
| 0 - 85 Gew.-% | Stearinsäure |
| 0 - 10 Gew.-% | Ölsäure |
| 0 - 90 Gew.-% | 12-Hydroxystearinsäure, |

wobei sich in der Summe immer 100 Gew.-% ergeben.

Geeignet ist hierbei reine (100 %ige) Hydroxystearinsäure sowie die technische Hydroxystearinsäure (ca. 90 %ig mit anderen Fettsäuren).

Bevorzugt enthält die Mischung langkettiger Fettsäuren

| | |
|---|---|
| 0 - 7 Gew.-% | Myristinsäure |
| 34 - 64 Gew.-% | Palmitinsäure |
| 64 - 45 Gew.-% | Stearinsäure |
| 0 -10 Gew.% | Ölsäure, |

wobei sich in der Summe immer 100 Gew.-% ergeben.

Besonders bevorzugt enthält die Mischung langkettiger Fettsäuren

| | |
|---|---|
| 0 - 5 Gew.-% | Myristinsäure |
| 40 - 60 Gew.-% | Palmitinsäure |
| 60 - 40 Ges.-% | Stearinsäure |
| 0 - 5 Gew.-% | Ölsäure, |

wobei sich in der Summe immer 100 Gew.-% ergeben.
Bevorzugt sind zusätzlich Bestandteile nativer oder synthetischer Fettsäuren enthalten.

Bevorzugt wird als aliphatisches Diamin Ethylendiamin eingesetzt.

Bevorzugt enthalten die Umsetzungsprodukte auch gesättigte und/oder ungesättigte Dicarbonsäuren.

Bevorzugt beträgt das Verhältnis von Mischungen langkettiger Carbonsäuren zu aliphatischen Diaminen zu Dicarbonsäuren (1,8 - 1,98) : 1,0 : (0,1 - 0,01).

Bevorzugt ergibt die Summe der Carboxylfunktionalität immer 2. Unter Carboxylfunktionalität wird die Gruppe -COOH und daraus abgeleitete Derivate wie -COOR mit R = Alkyl und -CONR2 mit R = H oder Alkyl verstanden.

Bevorzugt wird bei den Umsetzungsprodukten, die auch gesättigte und/oder ungesättigte Dicarbonsäuren enthalten, die Alkalizahl < 10 und die Säurezahl < 15 eingestellt.

Bevorzugt enthält bei den Umsetzungsprodukten, die auch gesättigte und/oder ungesättigte Dicarbonsäuren enthalten, die Mischung der langkettigen Fettsäuren

| | |
|---|---|
| 0 - 7 Gew.-% | Myristinsäure |
| 20 - 85 Gew.-% | Palmitinsäure |
| 85 - 45 Gew.-% | Stearinsäure |
| 0 - 10 Gew.-% | Ölsäure. |

wobei sich in der Summe immer 100 Gew.-% ergeben.

Bevorzugt enthält hierbei die Mischung langkettiger Fettsäuren

| | |
|---|---|
| 0 - 5 Gew.-% | Myristinsäure |
| 20 - 80 Gew.-% | Palmitinsäure |
| 80 - 20 Gew.-% | Stearinsäure |
| 0 - 10 Gew.-% | Ölsäure. |

wobei sich in der Summe immer 100 Gew.-% ergeben.

Bevorzugt wird bei den Umsetzungsprodukten, die auch gesättigte und/oder ungesättigte Dicarbonsäuren enthalten als Diaminkomponente Ethylendiamin in Kombination mit linearen und/oder cycloaliphatischen Diaminen eingesetzt.

Bevorzugt enthält diese Kombination
50 bis 100 Gew.-% Ethylendiamin und
0 bis 50 Gew.-% lineare und/oder cycloaliphatische Diamine.

Besonders bevorzugt enthält die Kombination
95 bis 99,99 Gew.-% Ethylendiamin und
0,01 bis 5 Gew.-% lineare und/oder cycloaliphatische Diaminen.

Bevorzugt werden als Diaminkomponente Ethylendiamin in Kombination mit linearen oder cycloaliphatischen Diaminen wie Hexamethylendiamin oder TCD-Diamin (Tricycoldecandiamin) eingesetzt.

Bevorzugt enthält hierbei die Mischung langkettiger Fettsäuren

| | |
|---|---|
| 0 - 7 Gew.-% | Myristinsäure |
| 0 - 85 Gew.-% | Palmitinsäure |
| 0 - 85 Gew.-% | Stearinsäure |
| 0-10 Gew.-% | Ölsäure |
| 0 - 90 Gew.-% | 12-Hydroxystearinsäure. |

wobei sich in der Summe immer 100 Gew.-% ergeben.

Die Aufgabe wird auch gelöst durch ein Verfahren zur Herstellung von Umsetzungsprodukten aus Mischungen langkettiger Fettsäuren und aliphatischen Diaminen, dadurch gekennzeichnet, dass man für die Umsetzungsprodukte eine Alkalizahl < 10 und eine Säurezahl < 15 einstellt.

Die Erfindung betrifft schließlich auch die Verwendung der erfindungsgemäßen Umsetzungsprodukte als Modifizierungsmittel für Bitumen.

Für die Beispiele wurde der Einfluss der Zusammensetzung der Fettsäuren, die als Rohstoffe für die Herstellung des Amidwachses eingesetzt wurden, untersucht. Geprüft wurden Mischungen gesättigter Fettsäuren verschiedener Kettenlängen, der Einfluss von ungesättigten Fettsäuren und von Hydroxyfettsäuren in diesen Mischungen, der Einfluss von Dimerfettsäuren sowie die Variation der Aminkomponente.

Die Produkte wurden nach bekannten Verfahren hergestellt und in Abmischung mit Bitumen B80 3ppH (Shell, GFK, Miro) geprüft. Untersucht wurden die für die Verarbeitung und Qualität des Asphalts relevanten Größen Viskosität, Erweichungspunkt (Ring/Kugel, DIN 52011, EN 1427), Nadelpenetration und Kältebruch nach Fraaß (DIN 52012, EN 12593). Als Vergleichsbeispiele wurden Produkte aus Standardfettsäuremischungen und kommerziell verfügbare EBS-Produkte (Ethylenbisstearoyldiamin) getestet.

Es wurde überraschend gefunden, dass spezielle Kombinationen der Fettsäuren sowie gegebenenfalls noch Variationen bei der Diaminkomponente und der Zusatz von Dimerfettsäure eine Verbesserung gegenüber dem Stand der Technik bewirken.

### Beispiele

### Allgemeines Herstellungsverfahren

Die Fettsäure wird in der angegebenen Menge (flüssig) in einen 11 Druckreaktor eingefüllt. Der Reaktor wird geschlossen inertisiert und auf 140°C aufgeheizt. Bei dieser Temperatur wird das Amin zudosiert. Nach der Aminzugabe wird auf 200°C erhitzt und das Reaktionswasser abdestilliert. Der Druck im Reaktor wird dabei auf ca. 2 bar eingestellt. Nach Beendigung der Umsetzung wird auf 150°C abgekühlt und auf Atmosphärendruck entspannt und die Schmelze ausgegossen. Zur Charakterisierung werden Alkalizahl (DGF Einheitsmethode M IV 4), Säurezahl (DIN 53403) und Tropfpunkt (DIN 51801/2, ASTM D 127) nach den genannten bekannten Methoden bestimmt.

Bei den eingesetzten Fettsäuren und Fettsäuremischungen wurde die Zusammensetzung nach Säurezahl berechnet und über Gaschromatographie geprüft. Zum Vergleich wurden im Markt verfügbare und für diese Anwendung empfohlene Amidwachse eingesetzt. Die Fettsäurezusammensetzung der Handelsprodukte wurde über Gaschromatographie geprüft. Die Fraaß-Werte wurden in einer Mischung aus 3 Teilen Wachs und 97 Teilen Bitumen B80 ermittelt

**Tabelle 3: Beispielwachse und Vergleichsprodukte aus Ethylendiamin und Monocarbonsäuremischungen**

| Beispiel | 1 | 2 | 3 | 4 | 5 | 6 | 7 | 8 |
|---|---|---|---|---|---|---|---|---|
| Ethylendiamin | 1 | 1 | 1 | 1 | 1 | 1 | 1 | |
| Stearinsäure 98-100 | | | | 2 | | | | |
| Talgfettsäure 80/20 | | 2 | | | | | | |
| Talgfettsäure 70/30 | 2 | | 2 | | | | | |
| Palmitinsäure 98-100 | | | | | 2 | | | |
| Talgfettsäure 65/35 | | | | | | 1 | | |
| Talgfettsäure 60/40 | | | | | | | 2 | |
| Talgfettsäure 55/45 | | | | | | | | 2 |
| SZ | 5 | 5 | 5 | 10 | 9 | 3 | 8 | 9 |
| AZ | 5 | 5 | 5 | 5 | 5 | 105 | 7 | 5 |
| Tp | 144 | 144 | 144 | 144 | 146 | 126 | 144 | 144 |
| Fraß-Wert | -10-13 | -10-11 | -6-8 | -15-17 | -14-16 | -1720.- | -15.-18 | -15.-18 |

**Tabelle 4: Beispielwachse aus Ethylendiamin und Monocarbonsäuremischungen unter Zusatz von aliphatischen Diaminen**

| Beispiel | 9 | 10 | 11 | 12 | 13 | 14 | 15 | 16 |
|---|---|---|---|---|---|---|---|---|
| Ethylendiamin | 1 | 1 | 1 | 1 | 1 | 1 | 1 | 1 |
| Hexamethylendiamin | | 0,03 | 0,03 | | | 0,03 | | |
| TCD-Diamin | | | | 0,03 | 0,03 | | 0,02 | |
| Talgfettsäure 80/20 | | | | | | 2,06 | | |
| Talgfettsäure 70/30 | | | | 2,03 | | | | |
| Talgfettsäure 60/40 | | 1,96 | | | | | | |
| Talgfettsäure 55/45 | 1,87 | | 2,03 | | 1,96 | | | |
| Talgfettsäure 50/50 | | | | | | | 2,02 | |
| Ölsäure | 0,17 | 0,09 | | | 0,09 | | | |
| 12-Hydroxystearinsäure | | | | | | | | 2 |
| Säurezahl | 10 | 9 | 7 | 8 | 11 | 15 | 5 | 8 |
| Alkalizahl | 4 | 6 | 2 | 4 | 8 | 9 | 5 | 12 |
| Tropfpunkt | 136 | 138 | 139 | 138 | 136 | 138 | 142 | 140 |
| Fraß-Wert | -14.-16 | -15.-17 | -14.-16 | -15.-18 | -15.-17 | -13.-18 | -15.-18 | -14-16 |

**Tabelle 5: Beispielwachse aus Ethylendiamin und Monocarbonsäuremischungen unter Zusatz von aliphatischen Diaminen und/oder aliphatischen Dicarbonsäuren**

| Beispiel | 17 | 18 | 19 | 20 | 21 | 22 | 23 |
|---|---|---|---|---|---|---|---|
| Ethylendiamin | 1 | 1 | 1 | | | 1 | |
| Hexamethylendiamin | | | 0,04 | 0,05 | 1 | | 1 |
| TCD-Diamin | | | | | | | |
| Talgfettsäure 80/20 | | | | | | | |
| Talgfettsäure 70/30 | | | | 2 | | | |
| Talgfettsäure 65/35 | | | | | 1,82 | 1,82 | 1,82 |
| Talgfettsäure 60/40 | | | | | | | |
| Talgfettsäure 55/45 | 1,87 | 1,83 | 2,03 | | | | |
| Talgfettsäure 45/50 | | | | | | | |
| Ölsäure | | | | | | | |
| Hydroxystearinsäure | | | | | | | |
| Dimerfettsäure 1025 | | 0,08 | 0,05 | | | | |
| Adipinsäure | 0,07 | | | 0,05 | | | |
| Sebacinsäure | | | | | 0,09 | 0,09 | |
| Dodecandisäure | | | | | | | 0,09 |
| Säurezahl | 10 | 10 | 12 | 8 | 8 | 15 | 6 |
| Alkalizahl | 4 | 5 | 5 | 2 | 1 | 3 | 2 |
| Tropfpunkt | 151 | 138 | 136 | 159 | 149 | 180 | 148 |
| Fraß-Wert | -10..-13 | -17..-20 | -16...-20 | -16..-19 | -12..-14 | -11..-14 | -11..-13 |

Die Messwerte für den Kältebruch zeigen, dass der Charakter der Fettsäure und die Kettenverteilung in der Fettsäuremischung erheblichen Einfluss auf die Eigenschaften des Bitumens haben. Bei den reinen Fettsäuren liegen die Werte bei niedrigen Temperaturen, reine Fettsäuren sind aber wirtschaftlich unattraktiv, natürlich vorkommende Fettsäuremischungen wie Talgfettsäure gehärtet oder Palmkernsäure gehärtet führen zu dem schon beschriebenen Anstieg der Bruchtemperatur.

Erst beim Einsatz der erfindungsgemäßen Fettsäurezusammensetzungen oder durch den Zusatz von anderen aliphatischen Diaminen oder durch den Zusatz von aliphatischen Dicarbonsäuren zu Talgfettsäure entstehen bei der Umsetzung Produkte mit niedrigem Bruchwert in der Bitumenmischung. Eine überraschende Ausnahme ist beim Einsatz von Hydroxystearinsäure festzustellen, die sowohl rein als auch in Kombination mit Talgfettsäure niedrige Bruchwerte zeigt.

### Physikalische Prüfungen:

Drei Teile Wachs werden mit 97 Teilen Bitumen bei 180°C für 30 Minuten gemischt. Die flüssige Mischung wird vergossen. Mit Probemengen der Vergussmasse werden die Prüfungen durchgeführt. Die Ergebnisse der Prüfungen sind in den nachfolgenden Tabellen aufgeführt.

**Tabelle 6a: Kenndaten von Bitumenabmischungen mit 3 % Modifizierungsmittel aus Tabelle 3**

| Vergleichswachs | | | aus Beispiel Nr.7 | aus Beispiel Nr. 4 | aus Beispiel Nur. 5 | aus Beispiel Nr.21 | aus Beispiel Nr.22 | aus Beispiel Nr. 23 |
|---|---|---|---|---|---|---|---|---|
| | | B80 | Erfindung | Vergleich * | Vergleich * | Clariant | FACl | Clariant |
| Talgfettsäure-Zusammensetzung | | alleine | 60/40 | 98/2 | 2/98 | 70/30 | 65/35 | 70/30* |
| Viskosität mPas | Methode a | 100 | 40 | 60 | 45 | 55 | 55 | 50 |
| | Methode b | 80 | 50 | 60 | 50 | 50 | 60 | 50 |
| Erweichungspunkt | | 52 | 100 | 95 | 95 | 85 | 87 | 85 |
| Ring / Kugel °C | | | | | | | | |
| Nadelpenetration in 1/10 mm | | 75 | 42 | 39 | 41 | 45 | 43 | 48 |
| Kältebruch nach Fraaß °C ** | c | -17-..-19 | -14-..-15 | -15-..-17 | -13..-15 | -11-..-13 3 | -10-..-11 | -6-..-8 |

| | | | | | | | | |
|---|---|---|---|---|---|---|---|---|
| Vergleich* : Wachse aus reinen Rohstoffen zum Vergleich Kältebruch nach Fraaß °C ** : Versuchsreihe mit 5 Messpunkten, min + max Viskositäten Platte/Kegel bei 180°C/in mPas a = D: 100 1/s b = D: 300 1/s | | | | | | | | |

**Tabelle 6b: Kenndaten von Bitumenabmischungen mit 3 % Modifizierungsmittel aus Tabelle 4**

| Wachs aus Beispiel | | 9 | 10 | 13 | 15 | 16 |
|---|---|---|---|---|---|---|
| | | | Erfindung | Erfindung | | - |
| Viskosität mPas | a | 60 | 55 | 50 | 60 | 50 |
| | b | 60 | 65 | 60 | 60 | 60 |
| Erweichungspunkt Ring/Kugel | | 99 | 100 | 98 | 97 | 88 |
| Nadelpenetration in 1/10 mm | | 51 | 47 | 49 | 46 | 46 |
| Kältebruch nach Fraaß °C | c | -14-..-16 | -15-..-17 | -15..-17 | -15-..-18 | -14..-16 |

| | | | | | | |
|---|---|---|---|---|---|---|
| Kältebruch nach Fraaß °C ** : Versuchsreihe mit 5 Messpunkten, min + max Viskositäten Platte/Kegel bei 180°C/in mPas a = D: 100 1/s b = D: 300 1/s | | | | | | |

**Tabelle 6c: Kenndaten von Bitumenabmischungen mit 3 % Modifizierungsmittel aus Tabelle 5**

| Wachs aus Beispiel | | 18 | 21 | 19 | 20 | 22 | 23 |
|---|---|---|---|---|---|---|---|
| | | | Erfindung | Erfindung | | | |
| Viskosität mPas | a | 50 | 70 | 40 | 40 | 50 | 40 |
| | b | 50 | 65 | 50 | 50 | 60 | 50 |
| Erweichungspunkt Ring/Kugel °C | | 98 | 97 | 102 | 97 | 100 | 99 |
| Nadelpenetration in 1/10 mm | | 42 | 40 | 52 | 43 | 38 | 41 |
| Kältebruch nach Fraaß °C | | -17..-20 | -12-..-14 | -16..-20 | -16..-19 | -11..-13 | -11..-14 |

| | | | | | | | |
|---|---|---|---|---|---|---|---|
| Kältebruch nach Fraaß °C ** : Versuchsreihe mit 5 Messpunkten, min + max Viskositäten Platte/Kegel bei 180°C/in mPas a = D: 100 1/s b=D: 300 1/s | | | | | | | |

Eine anwendungstechnische Prüfung im Spurinnentest hat gezeigt, dass durch die Modifizierung der Kettenverteilung keine anwendungstechnischen Nachteile erkennbar sind.

Spurinnentest, Eindringtiefe in mm

| Wachs nach | unmodifiziert | Beispiel 1 | Beispiel 7 |
|---|---|---|---|
| Gussasphalt | 8 | 4 | 3,9 |
| Tragschicht | 3 | 0,8 | 0,8 |
| Splitmastix | 3,8 | 0,8 | 0,9 |
| Asphaltbinder | 5,3 | 1,2 | 1,1 |

### Bewertung:

Unmodifiziertes Bitumen hat eine hohe Viskosität, einen niedrigen Erweichungspunkt und eine hohe Nadelpenetrationshärte. Es bricht aber erst bei relativ niedrigen Temperaturen. Durch den Zusatz von ca. 3 % Amidwachs wird die Viskosität bei Verarbeitungstemperatur gesenkt, das Netzverhalten verbessert und der Erweichungspunkt erhöht. Beim Einsatz nichterfindungsgemäßer Produkte wird aber der Brechpunkt nach Fraaß deutlich zu höheren Temperaturen verschoben.

Setzt man dagegen Umsetzungsprodukte aus Mischungen langkettiger Fettsäuren und aliphatischen Diaminen (Amidwachse) gemäß der Erfindung ein, so bleiben die guten Effekte der Standardprodukte erhalten, der Brechwert wird aber wieder in den Temperaturbereich von nichtmodifiziertem Bitumen abgesenkt. Der Praxistest zeigt, dass durch die Veränderung keine Nachteile in der Belastbarkeit bei den Spurrinnen auftritt.

## Patentansprüche

1. Umsetzungsprodukte aus Mischungen langkettiger Fettsäuren und aliphatischen Diaminen mit einer Alkalizahl < 10 und einer Säurezahl < 15, wobei das Verhältnis von Mischungen der langkettigen Fettsäuren zu aliphatischen Diaminen 2 zu 1 beträgt und
die Mischung langkettiger Fettsäuren
| | |
|---|---|
| 0 - 7 Gew.-% | Myristinsäure |
| 0 - 85 Gew.-% | Palmitinsäure |
| 0 - 85 Gew.-% | Stearinsäure |
| 0-10 Gew.-% | Ölsäure |
| 0 - 90 Gew.-% | 12-Hydroxystearinsäure |
enthält, wobei sich in der Summe immer 100 Gew.-% ergeben.

2. Umsetzungsprodukte nach Anspruch 1, **dadurch gekennzeichnet, dass** die
Mischung langkettiger Fettsäuren
| | |
|---|---|
| 0 - 7 Gew.-% | Myristinsäure |
| 34 - 64 Gew.-% | Palmitinsäure |
| 64 - 45 Gew.-% | Stearinsäure |
| 0 - 10 Gew.-% | Ölsäure |
enthält, wobei sich in der Summe immer 100 Gew.-% ergeben.

3. Umsetzungsprodukte nach Anspruch 1 oder 2, **dadurch gekennzeichnet, dass** die
Mischung langkettiger Fettsäuren
| | |
|---|---|
| 0 - 5 Gew.-% | Myristinsäure |
| 40 - 60 Gew.-% | Palmitinsäure |
| 60 - 40 Gew.-% | Stearinsäure |
| 0 - 5 Gew.-% | Ölsäure |
enthält, wobei sich in der Summe immer 100 Gew.-% ergeben.

4. Umsetzungsprodukte nach einem oder mehreren der Ansprüche 1 bis 3, **dadurch gekennzeichnet, dass** Bestandteile nativer oder synthetischer Fettsäuren enthalten sind.

5. Umsetzungsprodukte nach einem oder mehreren der Ansprüche 1 bis 4, **dadurch gekennzeichnet, dass** als aliphatisches Diamin Ethylendiamin eingesetzt wird.

6. Umsetzungsprodukte nach einem oder mehreren der Ansprüche 1 bis 5, **dadurch gekennzeichnet, dass** sie gesättigte und/oder ungesättigte Dicarbonsäuren enthalten.

7. Umsetzungsprodukte nach einem oder mehreren der Ansprüche 1 bis 6, **dadurch gekennzeichnet, dass** das Verhältnis von Mischungen langkettiger Carbonsäuren zu aliphatischen Diaminen zu Dicarbonsäuren (1,8 - 1,98) : 1,0 : (0,1 - 0,01) beträgt.

8. Umsetzungsprodukte nach Anspruch 6 oder 7, **dadurch gekennzeichnet, dass** die Summe der Carboxylfunktionalität immer 2 ergibt.

9. Umsetzungsprodukte nach einem oder mehreren der Ansprüche 6 bis 8, **dadurch gekennzeichnet, dass** die Alkalizahl < 10 und die Säurezahl < 15 eingestellt wird.

10. Umsetzungsprodukte nach einem oder mehreren der Ansprüche 6 bis 9, **dadurch gekennzeichnet, dass** die Mischung langkettiger Fettsäuren
| | |
|---|---|
| 0 - 7 Gew.-% | Myristinsäure |
| 20 - 85 Gew.-% | Palmitinsäure |
| 85 - 45 Gew.-% | Stearinsäure |
| 0 - 10 Gew.-% | Ölsäure |
enthält, wobei sich in der Summe immer 100 Gew.-% ergeben.

11. Umsetzungsprodukte nach einem oder mehreren der Ansprüche 6 bis 10, **dadurch gekennzeichnet, dass** die Mischung langkettiger Fettsäuren
| | |
|---|---|
| 0 - 5 Gew.-% | Myristinsäure |
| 20 - 80 Gew.-% | Palmitinsäure |
| 80 - 20 Gew.-% | Stearinsäure |
| 0-10 Gew.-% | Ölsäure |
enthält, wobei sich in der Summe immer 100 Gew.-% ergeben.

12. Umsetzungsprodukte nach einem oder mehreren der Ansprüche 6 bis 11, **dadurch gekennzeichnet, dass** als Diaminkomponente Ethylendiamin in Kombination mit linearen und/oder cycloaliphatischen Diaminen eingesetzt wird.

13. Umsetzungsprodukte nach einem oder mehreren der Ansprüche 6 bis 12, **dadurch gekennzeichnet, dass** die Kombination
50 bis 100 Gew.-% Ethylendiamin und
0 bis 50 Gew.-% lineare und/oder cycloaliphatische Diaminen enthält.

14. Umsetzungsprodukte nach einem oder mehreren der Ansprüche 6 bis 13, **dadurch gekennzeichnet, dass** die Kombination
95 bis 99,99 Gew.-% Ethylendiamin und
0,01 bis 5 Gew.-% lineare und/oder cycloaliphatische Diaminen enthält.

15. Umsetzungsprodukte nach einem oder mehreren der Ansprüche 6 bis 14, **dadurch gekennzeichnet, dass** als Diaminkomponente Ethylendiamin in Kombination mit linearen oder cycloaliphatischen Diaminen wie Hexamethylendiamin oder Tricycoldecandiamin eingesetzt wird.

16. Umsetzungsprodukte nach einem oder mehreren der Ansprüche 6 bis 15, **dadurch gekennzeichnet, dass** die Mischung langkettiger Fettsäuren
| | |
|---|---|
| 0 - 7 Gew.-% | Myristinsäure |
| 0 - 85 Gew.-% | Palmitinsäure |
| 0 - 85 Gew.-% | Stearinsäure |
| 0 -10 Gew.-% | Ölsäure |
| 0 - 90 Gew.-% | 12-Hydroxystearinsäure |
enthält, wobei sich in der Summe immer 100 Gew.-% ergeben.

17. Verfahren zur Herstellung von Umsetzungsprodukten nach einem oder mehreren der Ansprüche 1 bis 16, **dadurch gekennzeichnet, dass** man für die Umsetzungsprodukte eine Alkalizahl < 10 und eine Säurezahl < 15 einstellt.

18. Verwendung von Umsetzungsprodukte nach einem oder mehreren der Ansprüche 1 bis 16 als Modifizierungsmittel für Bitumen.

## Claims

1. A reaction product of mixtures of long-chain fatty acids and aliphatic diamines having an alkali number of < 10 and an acid number of < 15,
where the ratio of mixtures of long-chain fatty acids to aliphatic diamines is 2 to 1 and the mixture of long-chain fatty acids comprises
| | |
|---|---|
| 0-7% by weight | of myristic acid |
| 0-85% by weight | of palmitic acid |
| 0-85% by weight | of stearic acid |
| 0-10% by weight | of oleic acid |
| 0-90% by weight | of 12-hydroxystearic acid, |
where the sum is always 100% by weight.

2. The reaction product as claimed in claim 1, wherein the mixture of long-chain fatty acids comprises
| | |
|---|---|
| 0-7% by weight | of myristic acid |
| 34-64% by weight | of palmitic acid |
| 64-45% by weight | of stearic acid |
| 0-10% by weight | of oleic acid, |
where the sum is always 100% by weight.

3. The reaction product as claimed in claim 1 or 2, wherein the mixture of long-chain fatty acids comprises
| | |
|---|---|
| 0-5% by weight | of myristic acid |
| 40-60% by weight | of palmitic acid |
| 60-40% by weight | of stearic acid |
| 0-5% by weight | of oleic acid, |
where the sum is always 100% by weight.

4. The reaction product as claimed in one or more of claims 1 to 3, wherein natural or synthetic fatty acids are present as constituents.

5. The reaction product as claimed in one or more of claims 1 to 4, wherein ethylenediamine is used as aliphatic diamine.

6. The reaction product as claimed in one or more of claims 1 to 5 in which saturated and/or unsaturated dicarboxylic acids are present.

7. The reaction product as claimed in one or more of claims 1 to 6, wherein the ratio of mixtures of long-chain carboxylic acids to aliphatic diamines to dicarboxylic acids is (1.8-1.98):1.0:(0.1-0.01).

8. The reaction product as claimed in claim 6 or 7, wherein the sum of the carboxyl functionality is always 2.

9. The reaction product as claimed in one or more of claims 6 to 8, wherein an alkali number of < 10 and an acid number of < 15 are set.

10. The reaction product as claimed in one or more of claims 6 to 9, wherein the mixture of long-chain fatty acids comprises
| | |
|---|---|
| 0-7% by weight | of myristic acid |
| 20-85% by weight | of palmitic acid |
| 85-45% by weight | of stearic acid |
| 0-10% by weight | of oleic acid, |
where the sum is always 100% by weight.

11. The reaction product as claimed in one or more of claims 6 to 10, wherein the mixture of long-chain fatty acids comprises
| | |
|---|---|
| 0-5% by weight | of myristic acid |
| 20-80% by weight | of palmitic acid |
| 80-20% by weight | of stearic acid |
| 0-10% by weight | of oleic acid, |
where the sum is always 100% by weight.

12. The reaction product as claimed in one or more of claims 6 to 11, wherein the diamine component used is ethylenediamine in combination with linear and/or cycloaliphatic diamines.

13. The reaction product as claimed in one or more of claims 6 to 12,
wherein the combination comprises
from 50 to 100% by weight of ethylenediamine and
from 0 to 50% by weight of linear and/or cycloaliphatic diamines.

14. The reaction product as claimed in one or more of claims 6 to 13,
wherein the combination comprises
from 95 to 99.99% by weight of ethylenediamine and
from 0.01 to 5% by weight of linear and/or cycloaliphatic diamines.

15. The reaction product as claimed in one or more of claims 6 to 14, wherein the diamine component used is ethylenediamine in combination with linear or cycloaliphatic diamines such as hexamethylenediamine or tricyclodecanediamine.

16. The reaction product as claimed in one or more of claims 6 to 15,
wherein the mixture of long-chain fatty acids comprises
| | |
|---|---|
| 0-7% by weight | of myristic acid |
| 0-85% by weight | of palmitic acid |
| 0-85% by weight | of stearic acid |
| 0-10% by weight | of oleic acid, |
| 0-90% by weight | of 12-hydroxystearic acid, |
where the sum is always 100% by weight.

17. A process for preparing reaction products as claimed in one or more of claims 1 to 16, wherein an alkali number of < 10 and an acid number of < 15 are set for the reaction products.

18. The use of reaction products as claimed in one or more of claims 1 to 16 as modifiers for bitumen.

## Revendications

1. Produits de transformation de mélanges d'acides gras à longue chaîne et de diamines aliphatiques présentant un indice d'alcalin < 10 et un indice d'acide < 15, le rapport des mélanges des acides gras à longue chaîne aux diamines aliphatiques étant de 2:1 et le mélange contenant les acides gras à longue chaîne
| | |
|---|---|
| 0-7% en poids | d'acide myristique |
| 0-85% en poids | d'acide palmitique |
| 0-85% en poids | d'acide stéarique |
| 0-10% en poids | d'acide oléique |
| 0-90% en poids | d'acide 12-hydroxystéarique |
la somme résultant toujours en 100% en poids.

2. Produits de transformation selon la revendication 1, **caractérisés en ce que** le mélange contient les acides gras à longue chaîne
| | |
|---|---|
| 0-7% en poids | d'acide myristique |
| 34-64% en poids | d'acide palmitique |
| 64-45% en poids | d'acide stéarique |
| 0-10% en poids | d'acide oléique |
la somme résultant toujours en 100% en poids.

3. Produits de transformation selon la revendication 1 ou 2, **caractérisés en ce que** le mélange contient les acides gras à longue chaîne
| | |
|---|---|
| 0-5% en poids | d'acide myristique |
| 40-60% en poids | d'acide palmitique |
| 60-40% en poids | d'acide stéarique |
| 0-5% en poids | d'acide oléique |
la somme résultant toujours en 100% en poids.

4. Produits de transformation selon l'une ou plusieurs des revendications 1 ou 3, **caractérisés en ce que** des constituants d'acides gras naturels ou synthétiques sont contenus.

5. Produits de transformation selon l'une ou plusieurs des revendications 1 ou 4, **caractérisés en ce qu'**on utilise comme diamine aliphatique de l'éthylènediamine.

6. Produits de transformation selon l'une ou plusieurs des revendications 1 ou 5, **caractérisés en ce qu'**ils contiennent des acides dicarboxyliques saturés et/ou insaturés.

7. Produits de transformation selon l'une ou plusieurs des revendications 1 ou 6, **caractérisés en ce que** le rapport de mélanges d'acides carboxyliques à longue chaîne aux diamines aliphatiques aux acides dicarboxyliques est de (1,8-1,98):1,0:(0,1-0,01).

8. Produits de transformation selon la revendication 6 ou 7, **caractérisés en ce que** la somme de la fonctionnalité carbonyle est toujours égale à 2.

9. Produits de transformation selon l'une ou plusieurs des revendications 6 à 8, **caractérisés en ce que** l'indice d'alcalin est réglé à < 10 et l'indice d'acide à < 15.

10. Produits de transformation selon l'une ou plusieurs des revendications 6 à 9, **caractérisés en ce que** le mélange contient les acides gras à longue chaîne
| | |
|---|---|
| 0-7% en poids | d'acide myristique |
| 20-85% en poids | d'acide palmitique |
| 85-45% en poids | d'acide stéarique |
| 0-10% en poids | d'acide oléique |
la somme résultant toujours en 100% en poids.

11. Produits de transformation selon l'une ou plusieurs des revendications 6 à 10, **caractérisés en ce que** le mélange contient les acides gras à longue chaîne
| | |
|---|---|
| 0-5% en poids | d'acide myristique |
| 20-80% en poids | d'acide palmitique |
| 80-20% en poids | d'acide stéarique |
| 0-10% en poids | d'acide oléique |
la somme résultant toujours en 100% en poids.

12. Produits de transformation selon l'une ou plusieurs des revendications 6 à 11, **caractérisés en ce qu'**on utilise comme composant diamine de l'éthylènediamine en combinaison avec des diamines linéaires et/ou cycloaliphatiques.

13. Produits de transformation selon l'une ou plusieurs des revendications 6 à 12, **caractérisés en ce que** la combinaison contient
50 à 100% en poids d'éthylènediamine et
0 à 50% en poids de diamines linéaires et/ou cycloaliphatiques.

14. Produits de transformation selon l'une ou plusieurs des revendications 6 à 13, **caractérisés en ce que** la combinaison contient
95 à 99,99% en poids d'éthylènediamine et
0,01 à 5% en poids de diamines linéaires et/ou cycloaliphatiques.

15. Produits de transformation selon l'une ou plusieurs des revendications 6 à 14, **caractérisés en ce qu'**on utilise comme composant diamine de l'éthylènediamine en combinaison avec des diamines linéaires ou cycloaliphatiques telles que l'hexaméthylènediamine ou la tricyclodécanediamine.

16. Produits de transformation selon l'une ou plusieurs des revendications 6 à 15, **caractérisés en ce que** le mélange contient les acides gras à longue chaîne
| | |
|---|---|
| 0-7% en poids | d'acide myristique |
| 0-85% en poids | d'acide palmitique |
| 0-85% en poids | d'acide stéarique |
| 0-10% en poids | d'acide oléique |
| 0-90% en poids | d'acide 12-hydroxystéarique |
la somme résultant toujours en 100% en poids.

17. Procédé pour la préparation de produits de transformation selon l'une ou plusieurs des revendications 1 à 16, **caractérisé en ce qu'**on règle pour les produits de transformation un indice d'alcalin < 10 et un indice d'acide < 15.

18. Utilisation des produits de transformation selon l'une ou plusieurs des revendications 1 à 16 comme agent de modification de bitumes.
